(19) Europäisches Patentamt

European Patent Office

Office européen des brevets

(11) Publication number : **0 523 928 A2**

(12) **EUROPEAN PATENT APPLICATION**

(21) Application number : 92306363.0

(22) Date of filing : 10.07.92

(51) Int. Cl.$^5$ : **C08G 18/77,** C08G 18/38, A61M 25/00

(30) Priority : 12.07.91 US 730190

(43) Date of publication of application :
20.01.93 Bulletin 93/03

(84) Designated Contracting States :
DE ES FR GB IT

(71) Applicant : CRITIKON, INC.
4110 George Road
Tampa Florida 33634 (US)

(72) Inventor : Wagener, Kenneth B.
5012 NW 15th Place
Gainesville, Florida 32605 (US)
Inventor : Hentschel, Peter
Unterer Giebelweg 6
W-8761 Laudenbach 6 (DE)
Inventor : Cambron, Ronald E.
2932 Pinewood Run
Palm Harbor, Florida 34684 (US)
Inventor : Brodowski, Walter
Neudorfer Strasse 8
W-8762 Amorback (DE)

(74) Representative : Mercer, Christopher Paul et al
Carpmaels & Ransford 43, Bloomsbury Square
London WC1A 2RA (GB)

(54) **Kink resistant, flexible, radiopaque polyurethane tubing and catheters formed therefrom.**

(57) Catheters formed from polyurethane containing sufficient halogenated moieties in the polymer structure to make the polyurethanes radiopaque. Such polyurethanes can be prepared by employing halogenated diols or halogenated diisocyanate reactants. In preferred cases, the catheters are optically transparent in addition to being radiopaque. Furthermore, the catheters contain virtually kink-free resistance, up to at least 85% recovery volumne flow after bending.

EP 0 523 928 A2

## Background of the Invention

Catheters are slender tubes widely employed in the medical field for insertion into body passages, vessels or cavities. They are employed for passing fluids, draining fluids, making examinations, etc.

It is generally desirable that catheters be radiopaque because it is often necessary to determine the precise location of a catheter within its host by X-ray examination. In addition, it would be advantageous if catheters were optically transparent so that the flow of fluids therethrough could be observed.

There has been extensive research over a long period to improve the properties of catheters, including the properties of X-ray opacity and optical transparency. This research is documented, for example, in the patent literature.

U.S. Patent No. 2,212,334 issued to Wallerich in 1940 describes early attempts to produce an optically transparent catheter having some radiopaque properties. In this patent, Wallerich describes the extrusion of a plastic cellulose material through a tubular molding die coupled with forcible injection of small quantities of X-ray opaque material at uniform brief intervals to vary the X-ray opacity of the extruded catheter at regular intervals.

U.S. Pat. No. 2,857,915 issued to Sheridan describes efforts to produce catheters which were normally transparent to visible light but having an integral continuous opaque stripe running along the length of the catheter. The polymers suggested by Sheridan included nylon, polyester, polyethylene, and vinyl.

The art of adding a radiopaque stripe to a catheter was further refined as described in U.S. Pat. Nos. 4,027,659 and 4,105,732 issued to Slingluff. Slingluff added a highly conductive material, as well as a radiopaque material, to the stripe running along the length of the catheter. Thus, the stripe could be employed for observing the catheter's location with X-rays and for electrically grounding the catheter to allow discharge of any electrostatic charges built up during use of the catheter.

In U.S. pat. No. 3,605,750, issued to Sheridan et al., catheters having radiopaque distal end portions are described. These are made X-ray opaque by fusing a plastic annulus containing X-ray opaque pigment onto a preformed catheter tube.

U.S. pat. No. 3,529,633 issued to Vaillancourt describes the many prior efforts to provide a catheter which was optically transparent and yet had radiopaque properties. In this patent, Vaillancourt suggests that catheters be formed from fluorinated polymers, such as polytetrafluoroethylene, having an adequate quantity of precipitated barium sulfate to provide X-ray opacity with a minor portion of the catheter or "window" remaining clear and transparent.

Greyson, in U.S. Pat. No. 3,608,555, suggests incorporating an X-ray opaque substance having an index of refraction close to that of the polymer to provide radiopacity with sufficient optical permeability to permit viewing of fluids within the catheter. Greyson also suggests that crystallization should be minimized for crystalline-forming polymers such as perfluorocarbon resins.

In U.S. Pat. No. 3,618,614, Flynn suggests multiwall surgical tubing having an inner relatively thick transparent tube encased in a relatively thin, visually transparent, outer shell containing a radiopaque material. Thus, X-rays pass through the lateral edges of the composite tube through a relatively long path at the side edges of the tubing while the central portion remains substantially transparent.

Flynn suggests the addition of certain radiopaque plasticizers to vinyl resins employed in the formation of medical-surgical tubing in U.S. pat. No. 3,645,955. These plasticizers include halogenated benzoates, such as alkyl, 2,5-diiodobenzoates, alkyl or alkoxyalkyl 2,3,4,6-tetraiodobenzoates, or mixtures thereof.

In U.S. Pat. No. 3,336,918, Jeckel describes the use of polyurethane coatings containing radiopaque metal powders such as tin, lead and bismuth, for use in catheters. It had previously been found that the addition of such radiopaque metals accelerated the urethane reaction, limiting their pot-life. The specific invention described by Jeckel in this patent is the use of small amounts of diglycolic acid to control or halt the catalytic action of the heavy metal powder on the urethane reaction, thereby lengthening the pot-life.

In U.S. Pat. Nos. 3,749,134 and 3,901,829, Slingluff describes yet further attempts to produce catheters which are optically transparent and radiopaque. In these patents, Slingluff suggests blending a small amount of a diol of tetrabromophthalic anhydride and a plasticizer with the thermoplastic resin employed in forming the catheter. The diol is distributed throughout the entire wall of the tubing rendering it radiopaque or, alternately, the diol can be limited to certain areas or zones or added in any desired pattern. The use of such diols and plasticizers is suggested with a wide variety of thermoplastic resins, such as polyethylene, vinyl polymers, nylon, flexible polyurethane, etc.

Goossens et al. suggest that optically clear radiopaque catheters can be formed from certain terpolymers in U.S. Pat. No. 4,182,787. These are terpolymers of polycarbonatepolydiorgansiloxane having carbonate, halocarbonate, and polydiorganosiloxane constituents.

U.S. Pat. No. 4,282,876 issued to Flynn describes still another polymer composition intended to produce a combination of optical clarity with radiopacity for catheters. The polymers described are polyurethane resins, alone, or combined with vinyl resins, and having alkyl or alkoxyalkyl 2,5-diiodobenzoates, 2,3,4,6-tetraiodobenzoates or mixtures thereof added to provide radiopacity.

Generally, the suggestions described above have involved combining a structural resin for the catheter with a second component, intended to affect the X-ray opacity, in a physical blend. Unfortunately, it has been discovered that such physical blends suffer from certain drawbacks.

For example, the material blended in to add radiopacity often can be leached from the material. In extreme cases, the material can be leached from the catheter and absorbed systemically by the host.

In certain instances, as exemplified by the use of added barium sulfate, the incorporation of the added material has resulted in the creation of physical non-uniformities in the polymer blend causing the walls of the catheter to be abrasive. As a result, insertion and removal forces are increased which, in some cases, may result in patient discomfort.

The Goossens et al. patent referred to above, while not suggesting a mixture but instead suggesting a polymer having the properties of radiopacity and optical transparency, suffers from still other drawbacks. For example, it has been found that the Goossens et al. polymer exhibits stiffness which is not dissipated when a catheter made from this material is inserted into the blood vessel of a host.

Cambron, et al., U.S. Pat. No. 4,722,344 relates to polyurethanes, and to catheters and other articles formed from the polyurethanes, which are radiopaque due to the incorporation of halogenated moieties into the polymer structure. In preferred embodiments, the polyurethanes are additionally optically transparent. These unique polyurethanes can be produced by employing halogenated polyols, halogenated isocyanates, or both, as polymerization reactants.

Polyurethanes produced according to Cambron et al. '344 retain the properties which have made polyurethanes particularly useful in the fabrication of medical devices, including catheters. Thus, they are biocompatible materials and materials which are known to soften at body temperatures. The polyurethanes can be thermoplastic polymers capable of being processed by conventional polymer techniques into shaped articles possessing outstanding mechanical properties including tensile strength, elongation at yield and flexural modulus.

Importantly, the polyurethanes of Cambron et al., are radiopaque because of structural units contained within the polymer itself. This eliminates the necessity to blend a material with the polymer to provide radiopacity. Thus, the disadvantage of physical blends are avoided. Because the radiopacity is obtained from halogenated moieties contained within the structure of the polymer, these moieties do not leach out during use of the catheter and do not create non-uniformities in the polymer blend.

In preferred embodiments, the polyurethanes are optically transparent in addition to being radiopaque. polyurethanes which are radiopaque and yet optically transparent provide a unique combination of desirable properties for catheters in a polymer noted for its biocompatibility and other advantageous properties for medical applications.

Yet, it is also desirable for the catheter material to meet a number of other requirements. First and foremost, the catheter must exhibit problem-free extrudability. This characteristic allows the catheter to be manufacturable. Second, and quite importantly, the catheter should be kink resistant. Roughly, the material must contain a kink resistance better than that of Teflon™. For instance, the tubing should recover at least 85% to 90% of original volumetric tubular flow after kinking. The tubing flexural modulus should be somewhere between 550 and 700 Mpa.

As previously mentioned, the tubing should be resistant to solvents, particularly isopropanol. As well, the material should be sterilizable. That is, the polymer should be resistant to the affects of ethylene oxide or gamma rays. As previously mentioned, the surface should be smooth, to alleviate any problems during intravenous application.

Background of the Invention

These and other objects of the invention are disclosed by catheters which are formed from polyurethanes containing sufficient halogenated moieties in the polymer to make the polyurethanes radiopaque. In particular, certain polyurethane compositions are specifically disclosed which result in catheters having excellent kink recovery properties, even though these polymers are of relatively high modulus (stiffness) characteristics. These new compositions specifically utilize trans geometic isomer forms of various cycloaliphatic diisocyanate component materials in the polymer compositions, in order to obtain the unique mechanical performance features of high kink recovery and high modulus which may be uniquely advantageous in many clinical applications and uses.

The invention will be better understood from the attached Detailed Description of the Drawings when taken in conjunction with the Description of the Invention.

## Detailed Description of the Drawings

The invention will be further described with reference to the following description of preferred embodiments of the invention when considered together with the attached drawings, in which:

Fig. 1 is a perspective view of an intravenous catheter assembly illustrating a catheter according to the present invention; and

Fig. 2 is a longitudinal cross-sectional view of the catheter assembly of Fig. 1.

## Detailed Description of the Invention

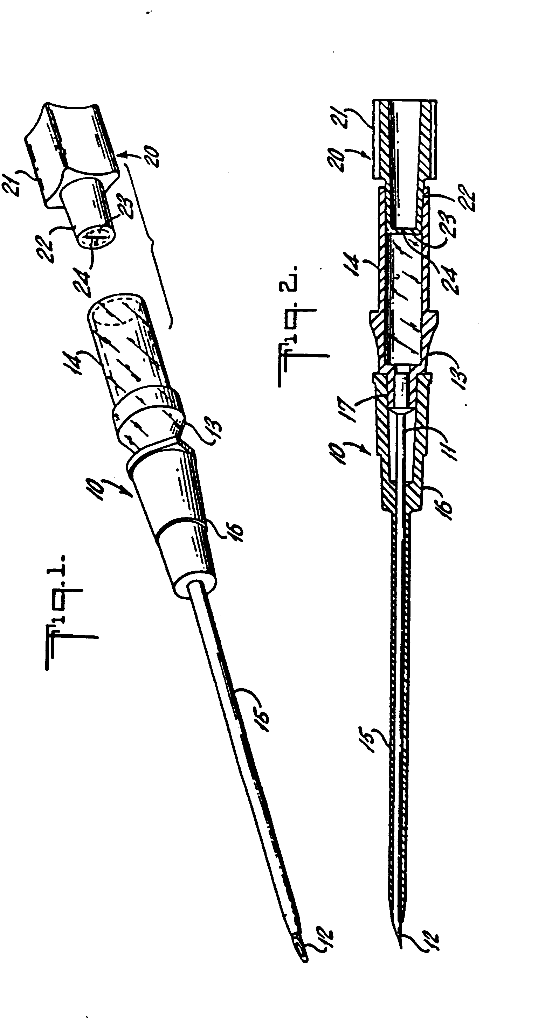

Referring to Figs. 1 and 2, an intravenous catheter assembly is shown generally at 10. The assembly comprises an introducer needle 11 which is in the form of a hollow hypodermic needle having a point 12 on one end thereof. Needle 11 is secured at its blunt end to a plastic hub 13 which has a transparent blood-detecting chamber 14 integral with its proximal end. The entire hub and blood-detecting chamber assembly can be molded in one piece from a suitable clear plastic material. Needle 11 serves the function of introducing a flexible plastic catheter 15 into a vein or other body vessel. Catheter 15 is attached to a hub 16 at its proximal end and hub 16 is adapted to be removably secured to a fitting 17 on the distal end of hub 13.

The plug shown generally at 20 in the drawing includes an enlarged gripping surface 21, a tapered neck portion 22 for insertion into the proximal end of blood-detecting chamber 14, and a diaphragm 23 having a diametrically oriented slit 24 formed therein. Plug 20 can be molded in one piece with diaphragm 23 formed at the distal end of the plug from a relatively thin portion of the plastic material. Slit 24 can then be formed in the diaphragm by cutting or other suitable procedure.

The size of slit 24 is not critical. However, it has been found to be desirable to form the slit in diaphragm 23 without the removal of any of the plastic material. This assures that air may be vented from the blood-detecting chamber but provides a seal against the passage of blood from the chamber. For purposes of illustration, slit 24 has been shown in the Figures in an enlarged condition so that it will be apparent that there is an opening in diaphragm 23.

To initiate the introduction of the needle 11 into a vein, the unit is fully assembled as shown in Fig. 2 with catheter 15 positioned over the needle 11, and with plug 20 firmly seated within the proximal end of blood-detecting chamber 14. The introduction of the needle point 12 into a vein will cause blood to flow through the hollow needle and into blood-detecting chamber 14. Air contained within the hollow needle and the blood-detecting chamber will be forced by the blood through slit 24 in plug 20 out into the atmosphere. Blood flowing into chamber 14 can then be detected by the operator through the transparent wall of the chamber. Because of the extremely small size of slit 24, blood will be retained within the chamber and not permitted to pass through the axial opening in plug 20. When it is desired to attach an administration set or other device to the catheter hub, it is only necessary to withdraw the needle from the catheter and, thereby, expose the open female luer end of hub 16 for the appropriate male fitting.

Catheter 15 is formed from a polyurethane which is radiopaque, and preferably optically transparent. The preparation of such polyurethanes will now be described.

In general polyurethanes are condensation products of reactions between diisocyanates (isocyanate compounds having a functionality of two) and polyols, such as diols. polyurethane chemistry is well understood in the art. See, for example, Saunders, J.H. and Frisch, K.C., "Polyurethanes, Part I", Interscience Publishers, New York (1962).

Suitable diisocyanate compounds necessary for achieving the objectives of this invention are very specific and are limited to (trans) geometric isomer forms of cycloaliphatic diisocyanate materials. Examples of trans-cycloaliphatic diisocyanates include trans-1,4 cyclohexandiisocyanate, trans,trans-dicyclohexylmethane-4,4′ diisocyanate, and trans-1,3-isophoronediisocyanate.

Suitable polyol compounds which may be employed in achieving the objectives of this invention include low and high molecular weight diols, and combinations thereof. Examples of low molecular weight diols include ethylene glycol, propylene glycol, butanediol, pentanediol, hexanediol, heptanediol, and isomers of the same. Examples of high molecular weight diols include polyethylene glycols, polypropylene glycols, polytetramethylene glycols, polyester diols, polycarbonatediols and copolymers and mixtures thereof.

Suitable halogen-containing diol compounds which may be employed in achieving the objectives of this invention include chlorine, bromine, and iodine substituted low molecular weight aliphatic, cycloaliphatic and aromatic glycols, polyesterdiols, polycarbonatediols, and polyetherdiols. Examples include mono-, di-, and tribromo neopentyl glycol compounds and dimers of the same; Bis-(2-hydroxyethyl)-tetrabromobisphenol A (PHTA 4-diol marketed by Great Lakes Chemical, West Lafayette, IN), and mixed ester diol adducts of tetrabromophthalic anhydride (Great Lakes or Saytex™ RB-79 Diol (Ethyl Corp.)).

Other suitable halogen moieties which may be employed in achieving the objectives of this invention may include halogenated trans-cycloaliphatic diisocyanate reactants. Examples include di-, tri-, and tetrabromo trans-1,4-cyclohexanediisocyanate; and di-, tri-, and tetrabromodicyclohexylmethane-trans, trans-4,4′-diisocyanate and di-, tri-, and tetrabromo-trans-1,3-isophoronediisocyanate.

The halogen containing polymerization reactants are employed in an amount which provides the final polyurethane with sufficient halogenated moieties to make the polymer radiopaque. The term "radiopaque" is used herein to mean that catheters and other shaped articles produced from the polyurethanes can be detected by customary X-ray examination procedures.

In addition to possessing outstanding radiopacity, it has been found that the polyurethanes of this invention can also be optically transparent. Such materials are particularly preferred for use in catheters because of this unique combination of desirable properties. The term "optical transparency" is used herein to mean a material which, when formed into a catheter or other shaped article, will allow the presence of blood or other fluids to be visible from outside the catheter under normal lighting conditions. For example, a polyurethane is optically transparent if such a material, when formed into a tube having a wall thickness of about 0.01 inches or less, allows the passage of blood therein to be observed by the naked eye from outside the tube under normal lighting conditions.

Polyurethanes produced according to this invention can be thermoplastic or thermoset polymers. Thermoplastic polyurethanes are often preferred because they can be melt-processed by conventional polymer techniques, such as injection molding, extrusion, etc. Thermoplastic polyurethanes are essentially linear polymers having no significant cross-linking.

Catheters, according to the invention described herein, are useful in medical product applications. Such catheters can be used, for example, for arterial, intravenous and central line vascular catheters, cardiovascular catheters, such as balloon thermodilution catheters, balloon wedge pressure catheters, Berman and angiographic catheters and balloon pacing catheters. Tubing formed from the polyurethanes containing halogenated moieties according to this invention can additionally be employed in other in vivo applications, including enteral feeding. The polyurethanes of this invention can also be employed in additional applications wherein the unique combination of radiopacity and optical transparency is desired or required.

## Radiopaque polyurethane formed from Trans-cyclohexane 1,4-diisocyanate(t-CHDI)

For medical applications, polyurethanes from aliphatic diisocyanates are preferred due to the toxicological harmlessness of their degradation products. It has therefore been attempted to formulate radiopaque polyurethanes with trans-cyclohexane 1,4-diisocyanate(t-CHDI). The remaining components of the formulation were selected according to the following criteria. The macrodiol must cause the t-CHDI combination to become suitable for sufficient resistance to thermo-oxydative degradation. However, it also must be hydrolysis resistant. In this regard, it is necessary to select a soft segment such as polyhexane-1,6-diolcarbonate (Desmophen 2020, available from Bayer AG Germany and Permuthan™ KM-10-1733, available from Permuthane Corp., USA) as the soft segment. Other possible combinations may be its sister chemical Desmophen 2028i (Bayer) or Formrez E-65-56 from Witco Co., USA.

The chain extender chosen must have, in general, a high hard segment melting point, in general higher than the polyurethanes used in the Cambron et al., patent. To get into the thermoplastic range it is necessary to select long chain, mobile compounds such as chain extender diols which reduce the density of the hydrogen bridge and crystallization of the hard segment. Alkoxilated bisphenol-A derivatives (dianols) have in the past proven to be most suitable chain extenders for transparent, thermoplastic t-CHDI-polyurethanes. Their softening temperatures are considered sufficient for the intended application as catheter material. Also, tetrabromodianol has already been successfully tested as a chain extender. Tetrabromodianol is commercially obtainable from Great Lakes Chemical Corporation, West Lafayette, Indiana.

In those cases where catalyst is necessary for the production of a t-CHDI-transparent polyurethanes (for instance, in the reaction extrusion process) it is most important to ensure that its toxicity is as minimal as possible if the product is used in the medical field. Organo-bismuth compounds have a distinctly lower toxicity than tin, lead, or mercury compounds which are traditionally used as polyurethane catalysts. In addition, organo-bismuth compounds catalyze the hydrolytic and thermal degradation of the polyurethanes to a lesser extent. It would therefore be advisable to use the bismuth catalyst Coscat 83 (Cosan Chemical Corp.) if necessary.

Thus, the resulting material takes on a composition such as a pair of hard segments, (the t-CHDI material in combination with a butane diol or a tetrabromodianol) sandwiching a soft segment (generally Desmophen 2020 or other materials such as Elastophen 2002 available from BASF). This soft segment polycarbonate material generally has a molecular weight of about 2000 and exhibits good melting behavior.

The typical batch process for formulating a large batch such as 100 Kg pots of these formulations takes place as follows. The molten soft segment, such as Desmophen 2020, has its water component removed by boiling at 100°C in a vacuum. This molten soft segment is then cooled to about 70°C. Simultaneously, the solid tetrabromodianol is melted at roughly 125 to 130°C. It has its water component removed in a vacuum. Both the molten soft segment, the melted tetrabromodianol, and the butane dianol which will combine in the hard segment are added into the mixture with the molten soft segment. This mixture is mixed at roughly 90°C. Once the mixture is complete, the solid cyclohexane diisocyanate is added at roughly 70°C. The final mixture is allowed to cool and it is cast within 30 minutes time. (It is to be noted that the pot life time of the mixture is quite short.) Then, the mixture is cured in an oven at about 110°C for 16 hours, so that the polyurethane will have sufficient molecular weight to be extrudable and drawn into the catheter tubing material.

EXAMPLE I

The invention is further illustrated by the following example. All polymer composition ingredients are expressed in relative molar level quantities. In a 50 liter stirred stainless steel vessel, the following molten ingredients were mixed, solubilized and dried under vacuum at 100°C, then cooled to 80°C;

| | Parts (molar levels) |
|---|---|
| Polycarbonatediol poly-1,6-hexanecarbonate (2000 mol. wt.) | 3.0 |
| Copolyesterdiol (poly-neopentylene 1,6-hexyleneadipatediol, 2000 mol. wt.) | 3.0 |
| Tetrabromodianol Bis-(2-hydroxyethyl)-tetrabromobisphenol A | 21.0 |
| 1,4-butanediol | 3.0 |

To the above premix, while rapidly stirring, was then added the diisocyanate component material, trans-1,4-cyclohexanediisocyanate (t-CHDI) at 31.5 molar parts. After being completely solubilized and being thoroughly mixed, the mass was cast into teflon coated trays. The cast material was then cured in an oven at 110°C for period of 16 hours. The resultant polyurethane was removed from the casting trays and granulated and dried prior to melt extrusion into small bore catheter tubing suitable for mechanical properties characterization testing. The test material was both radiopaque and optically transparent.

The extruded tubing characterization kink performance recovery and modulus properties of this product are shown in Table I. The Table also shows comparison properties of a polyurethane produced under earlier teachings of the Cambron et al., patent, U.S. Patent No. 4,722,344.

## TABLE I

### Extruded Tubing Properties Characterization

| Diisocyanate Component | Tubing Flex.* Modulus (psi) | Tubing Flow Recovery** after Kinking |
|---|---|---|
| Pure trans Isomer (t-CHDI) | 117,000 | 98% |
| Mixed cis/trans Isomer (cis/trans-HMDI) | 217,000 | 67% |

\* **Tubing Flexural Modulus**:
The test method is based on flexural modulus procedure as defined in ASTM D-790.

\*\* **Tubing Flow Recovery after Kinking**:
The test method involves determining the percentage flow recovery of water at 1 meter of head pressure and 25°C through a catheter tube at 3 minutes after the tube had been kinked.

Thus, it can be seen that the modulus of the tubing is quite high, that is, above 100,000 psi or 750,000 $N/mm^2$. Also, and more importantly, the kinking resistance is quite different for the two materials. The tubing now has a kink flow resistance of 97%, after kinking. The previously disclosed materials have kink flow resistance of only 67%. While diisocyanates may be thought of as having a high flex modulus, it is certainly not clear that they would be capable of such flexibility as derived by this invention. In fact, the teachings of the Cambron et al. 4,722,344 patent describe inflexible materials quite different from those of the current invention.

In view of the teachings herein, those skilled in the art will recognize, or will be able to develop using no more than routine experimentation, many equivalent specific embodiments of the invention described herein. For instance, one could envision capably using trans-1,3-isophoronediisocyante or trans,trans-dicyclohexylmethane -4,4'-diisocyante as satisfactory trans-CHCl alternatives. Such equivalents are intended to be encompassed by the following claims.

## Claims

1. A polyurethane containing sufficient halogenated moieties in the polymer structure to make the polyurethane radiopaque and to make a tube formed from the polyurethane kink-resistant to about 85%.

2. The polyurethane of claim 1, which contains at least 10 weight percent halogen.

3. The polyurethane of claim 1 or claim 2, which contains said halogenated moieties in its backbone.

4. The polyurethane of any one of claims 1 to 3, wherein said backbone includes a halogenated diisocyanate.

5. The polyurethane of claim 4, wherein the halogenated diisocyanate is a halogenated trans-cycloaliphatic diisocyanate.

6. The polyurethane of claim 5, which is the reaction product of the halogenated trans-cycloaliphatic diisocyanate with: a low molecular weight diol; a high molecular weight diol; or a combination of both.

7. The polyurethane of claim 5 or claim 6, wherein the diisocyanate is one of the family of bromine trans-1,4-cyclohexane diisocyanates.

8. The polyurethane of claim 5 or claim 6, wherein the diisocyanate is one of the family of bromine dicyclohexylmethane trans, trans-4,4'-diisocyanates.

9. The polyurethane of any one of claims 1 to 8, which contains a brominated chain extender in its backbone.

10. The polyurethane of any one of claims 1 to 9, which is optically transparent.

11. A tube formed from the polyurethane of any one of claims 1 to 10.

12. A kink-resistant catheter formed from the polyurethane of any one of claims 1 to 10.

Fig.1.

Fig.2.